# EUROPEAN PATENT SPECIFICATION

(11) **EP 3 942 106 B1**
(45) Date of publication and mention of the grant of the patent: **24.01.2024**
(21) Application number: 20721826.4
(22) Date of filing: 20.03.2020
(51) Int. Cl.: D21B 1/36

(54) **STEAM-EXPLOSION EQUIPMENT AND METHOD FOR TREATMENT OF ORGANIC MATERIAL**
DAMPFEXPLOSIONSANLAGE UND VERFAHREN ZUR BEHANDLUNG VON ORGANISCHEM MATERIAL
ÉQUIPEMENT DE VAPOCRAQUAGE ET PROCÉDÉ DE TRAITEMENT DE MATIÈRE ORGANIQUE

(30) Priority: 20.03.2019 IS 50258
(43) Date of publication of application: 26.01.2022
(73) Proprietor: YMIR Technologies ehf., 104 Reykjavík (IS)
(72) Inventor: INGOLFSSON, Oddur, 200 Kopavogur (IS); MATTHIASSON, Asgeir, 105 Reykjavik (IS); INGOLFSSON, Sigurdur, 200 Kopavogur (IS)
(74) Representative: Arnason Faktor
(86) International application number: PCT/IS2020/050009
(87) International publication number: WO 2020/188606

(56) References cited:
- WO-A1-2016/101076
- US-A1- 2013 264 264

## Description

### FIELD OF INVENTION

The invention relates to equipment and methods for semi-continuous steam-explosion and hydrolysis of organic material as advantageous pre-treatment prior to further processing for the production of value-added products from such material.

### TECHNICAL BACKGROUND

Steam explosion of biomass is a pre-treatment process that opens up the fibers, and makes the biomass polymers more accessible for subsequent processes, i.e. fermentation, hydrolysis or densification processes. Biomass materials such as wood are composite materials with high mechanical strength. The major components are cellulose, embedded in a matrix of lignin and hemicelluloses. Together they form tightly packed cellular structures (fibers) that form fiber bundles, and are the base for biomass tissues such as wood. Their natural function is to bear high mechanical loads, and to resist chemical and enzymatic degradation through microorganisms. Steam explosion has been shown to be a valuable and important technology to open up the biomass fibers, to improve the recovery of sugars and other useful compounds from biomass. It has also been proposed as pre-treatment process for the production of solid biofuel pellets to increase the calorific value, and to improve the pelletizing properties of the biomass.

Steam explosion was introduced and patented as a biomass pre-treatment process in 1926 by Mason et al. (1926). The patent describes a steam explosion process for the pre-treatment of wood. In this process, wood chips are fed from a bin through a screw loading valve in a masonite gun. The chips are then steam heated at a temperature of about 285°C and a pressure of 3.5 MPa for about 2 min. The pressure is increased rapidly to about 7 MPa (70 bar) for about 5 s, and the chips are then discharged through restricted orifices (slotted port) and explode at atmospheric pressure into a pulp.

In general, steam explosion is a process in which biomass is treated with hot steam (180 to 240°C) under pressure (1 to 3.5 MPa) followed by an explosive decompression of the biomass that results in a rupture of the biomass fibers rigid structure. The sudden pressure release defibrillates the cellulose bundles, and this result in better accessibility of the cellulose for enzymatic hydrolysis and fermentation. Depending on residence time and temperature, steam explosion can result in anything from small cracks in the wood structure, to total defibrillation of the wood fibers.

WO 2016/101076 discloses a continuous-flow steam explosion reactor for pre-treatment of organic material which is part of a system for producing non-fossil based synthetic hydrocarbons.

Both batch operated and continuous mode steam explosion reactors are known in the art, but specifically continuous steam explosion instrumentation is technically very challenging. Improved continuous-flow steam explosion reactors with improved efficiency and economy, such as by maintaining a high-pressure in continuous operation, increasing energy efficiency and reducing instrumental strain would be much appreciated.

### SUMMARY OF THE INVENTION

The steam explosion reactor of the present invention is defined in the claims and introduces modifications and advantages over the prior art. The steam explosion reactor effectuates abrupt disintegration of the unyielding structure of compact fibre materials making them accessible to efficient hydrolysis and subsequent decomposition such as through fermentation. The steam explosion unit further serves to effectuate hydrolysis and/or saponification of introduced organic waste material, such as celluloses, lignin, proteins and fats/oils, as well as extraction of soluble hydrocarbons, amino acids or peptides and fatty acids and salts thereof from the waste stream. The steam explosion pre-treatment process serves multiple purposes *i)* rupture of the structure of compact fiber material, *ii)* at least partial decomposition of low bioavailability material such as e.g., lignin and cellulosic material, *iii)* pre-hydrolysis of organic material, *iv)* aqueous extraction of nutrition from solid substrate and *v)* sterilization of all material.

The sterilization in the process is very advantageous as it widely enhances the utility for downstream products. Currently it is a challenge to effectively use certain waste streams from e.g. slaughterhouse facilities or fish or food processing plants, where waste can be contaminated with undesired bacteria, as well as household waste that often contains a significant amount of used diapers with urine and feces. The steam explosion treatment, in addition to the fragmenting and hydrolysis of material, provides desired sterilization. In many countries and regions use of slaughterhouse waste, food waste or other potentially bacterial waste such as for composting requires sterilization by autoclaving or the like. The present system provides such necessary sterilization of compost material, which accordingly is more acceptable and a higher value product.

In its most basic implementation the current invention relates to a steam explosion reactor that comprises a loading section, a high-pressure retention section, a pressure relief section and a discharge section, wherein said high-pressure retention section comprises at least one adjustable-speed conveyor for transporting said stream of material through said section. When run under alkaline conditions, the steam explosion reactor can further optionally comprise at least one integrated carbon dioxide scrubbing unit that can advantageously be configured to operate simultaneously with the steam explosion reactor.

The steam explosion reactor of the present invention comprises steam provision means for providing steam at least into the high-pressure retention section, and in some embodiments steam is also provided to the loading section and/or the pressure relief section. In advantageous embodiments the steam explosion reactor further comprises gas provision means that are connected to a source of pressurized gas for providing pressurized gas into selected compartments of the reactor. The pressurized gas can comprise pressurized air, but in some embodiments alternative gases can be used, or pressurized air supplemented with other gaseous compound such as in particular carbon dioxide, as will be further described herein. The pressurized gas can be used to supplement at least part of steam for generating high pressure. In some embodiments pressurized gas is provided to the high-pressure retention section. In other embodiments pressurized gas is additionally or alternatively provided to one or more of the loading section and the pressure relief section.

Accordingly, an aspect of this invention relates to equipment and methods for pre-treatment of organic material by means of steam explosion and hydrolysis, as advantageous treatment prior to further processing for the production of value-added products from such materials.

The continuous or semi-continuous steam explosion process and apparatus is intended for the treatment of organic material for promotion of its subsequent conversion to value-added products. The organic material treated in the steam explosion process can be of different origin and composition. The system and process of the present invention is generally directed to organic waste, or waste comprising mostly organic matter, which may or may not be pre-sorted, such as but not limited to organic waste from general household waste. Accordingly, the waste material received for treatment can comprise but is not limited to one or more, or any combination, of the following: Household waste, slaughterhouse waste, food industry waste, fish industry waste, waste from the vegetable oil and the fish oil industry, sewage sludge, sewage grease and oils, agricultural waste such as wheat-straw or other straw, rice husk, soybean curd residue, and grass and animal manure, as well as garden waste and waste wood.

Value-added products derived from the product stream produced in the steam explosion of the invention include methane produced through anaerobic digestion, ethanol produced through fermentation, soil-improving compost and biodiesel. These products or a combination of two or more of these products may be realized from different fractions of the steam-exploded material, subject to the separation process applied and the nature of the feed.

The steam explosion instrument may also be used for pre-treatment of raw material for paper production, for example, for pulping of wood and alternative raw material for paper production or other use.

When operating the steam explosion under alkaline conditions, the aqueous alkaline extract and the solid substrate are subsequently preferably lowered in pH in an integrated carbon dioxide scrubber as described herein below.

Prior to introduction to the high-pressure retention section, incoming material is preferably fed through a pre-treatment section that can comprise a wetting and mixing section, where water can be added to the stream, to obtain desired solid to liquid ratio and the material is mixed, and the pH can be affected, in particular for subsequent alkaline steam explosion, in which case alkaline solution is mixed into the stream in the wetting and mixing section. The wetting and mixing section preferably comprises a top-fed conveying mixer and wetting armature, such as one or more wetting nozzles. The exit port of the wetting and mixing section is connected via the loading section to the high-pressure retention section, such as with a valve assembly as described herein. In useful embodiments the loading section comprises a rotary dosing valve and more preferably a serial combination of a rotary dosing valve and a high-pressure rotary discharge valve. In this embodiment the rotary dosing valve ensures that a suitably sized dose of substrate is loaded into a compartment of a high-pressure rotary discharge valve before it is transferred to the high-pressure retention section. In another embodiment, a separate loading compartment is provided between the wetting and mixing section and high-pressure retention section. This compartment being confined by high-pressure entrance and exit valves as further described in detail herein below with reference to specific embodiments but applicable generally to the invention. The connection from the wetting and mixing section to the high-pressure retention section of the steam explosion reactor ensures that material can be continuously or semi-continuously transported from the ambient pressure wetting and mixing section to the high-pressure retention section, while the high-pressure is being continuously maintained in the latter.

In the high-pressure retention section, the loaded substrate is conveyed with suitable means from the loading point to the exit point such as but not limited to by means of an adjustable speed conveyor or mixing conveyor, preferably a screw conveyor or a mixing screw conveyor. The high-pressure retention section is equipped with at least one and preferably two or more steam injection ports and rotary valves or separate transfer compartments at the loading and releasing ends respectively, meaning that there can be at least one rotary valve but preferably two rotary valves at one end and at least one transfer compartment at the other end, or at least one but preferably two rotary valves at each end or at least one transfer compartment at each end. These are preferably each equipped with steam injection ports and pressure relief ports as appropriate and are advantageously synchronized such that pressure drop in the high-pressure retention section is minimal during operation, and retention time in the high-pressure retention section is adjustable over a wide range, by adjustment of the conveying speed which is synchronized with the loading and relief mechanism of the loading and releasing valves/compartments. The synchronization is advantageously controlled via a control unit such as a PLC system.

The steam explosion unit further comprises in some embodiments a grinding and/or homogenization unit that is arranged upstream of the wetting and mixing section feeding the continuous-flow steam explosion reactor. The grinding/homogenization unit can be of any suitable mechanical type, known to the skilled person, for grinding, and/or shredding or the like mechanical treatment of incoming material prior to further processing according to the invention.

It is particularly advantageous to operate the steam explosion treatment in continuous or semi-continuous fashion, this involves subjecting introduced material, preferably in alkaline or acidic solution, for the promotion of hydrolysis in the process, to high pressure and temperature, and discharging the material via sudden pressure-drop such as through a high-pressure rotary relief valve, or separate compartment. Suitable arrangements are described herein below, such that the high-pressure retention section is maintained at a high operating pressure and temperature, while substrate material is continuously or semi-continuously being introduced and discharged without significant pressure or temperature relief of the high-pressure retention section. This increases efficiency and reduces energy demands as compared to batch-operated steam explosion.

Further reduction in energy demand is achieved in some embodiments by assisting the steam explosion process by partly substituting the high-pressure steam generally required for this process with pressurized gas, such as typically pressurized air, as mentioned above. In such a configuration the desired temperature is generally achieved through steam injection, but the desired pressure can be achieved through the injection of pressurized gas such as air. This may be applied to all high-temperature/high-pressure sections of the steam explosion equipment or may be confined to the pressure relief section of the steam explosion equipment as described herein below.

For the promotion of hydrolysis and structural disintegration of the subject material in the steam explosion process the pressurized air may in some embodiments contain a partial pressure of CO₂ preferably a high partial pressure of CO₂ relative to the attainable partial pressure of CO₂ at the temperature and pressure range in the respective section/chamber into which the air mixture is provided. This embodiment serves to promote CO₂ penetration into the subject material and promoting internal disintegration through acid promoted hydrolysis and further supporting the mechanical disintegration of said material in the pressure relief step of the process.

As mentioned above, the steam explosion reactor of the invention comprises a pressure relief section. The term pressure relief section refers generally to the section of the reactor assembly where high pressure of the processed material is released, resulting in an explosive disruptive reaction. As further explained below, in some embodiments the pressure relief section comprises a dual valve assembly used for discharging material from the high-pressure retention section, such as with a rotary dosing valve and a rotary discharge valve, where the latter releases the substrate to a region of lower pressure (discharge section), such that the steam explosion takes place from the releasing compartment of the valve and the adjoining space beneath that receives the stream of material. In other embodiments the pressure relief section constitutes a separate chamber separated from the high-pressure retention section and the lower pressure receiving section/discharge section by high-pressure valves, the valves in such a configuration can be independently select from but are not limited to floating ball valves, rotary ball valves, knife gate valves, and slide gate valves.

In other embodiments the pressure relief section comprises a chamber confined by at least one loading valve, a pressure relief valve and at least one exit valve. In these embodiments the chamber is loaded from the side through the loading valve, pressure is relieved from above the loading port through a pressure relief valve and the chamber is discharge from its bottom through the exit valve. The chamber is suitably sized to receive an amount of substrate within a pre-determined range, and to accommodate the pressure relief steam explosion action. In useful embodiments the pressure relief valve is connected to an upper portion of said chamber and a relief conduit extending from said pressure relief valve for receiving escaping steam and/or gas. In these embodiments, In these embodiments, described herein below, the explosive pressure release is upwards, and gas and steam are released through a condenser for heat recovery. The substrate material, on the other hand, does not expand beyond the confinement of the pressure relief chamber, above which the pressure relief valve is placed. This embodiment is advantageous, as solid material does not transfer through the relief valve in the explosion step, thus considerably reducing the potential for sealing problems due to abrasion or through solid particles damaging the sealings. This embodiment is also advantageous as instrumental erosion caused by the high velocity solid material in the pressure relief section is minimized. In this embodiment the subject material is discharged from the bottom of the pressure relief chamber through the exit valve for further processing in a separate step after the steam explosion has taken place and the pressure is substantially lowered. In some of these embodiments a splash-guard such as but not limited to a mesh, a flange, perforated plate or cone, is arranged below the pressure relief valve to prevent or minimize substrate material coming into contact with the pressure relief valve. In some embodiments, pressurized gas injection means are installed in an upper portion of the chamber for injecting gas (such as air) after pressure release, to accelerate discharge of the steam-exploded material through the exit valve.

Accordingly, an aspect of the invention provides a continuous-flow or semi-continuous flow steam explosion reactor for pre-treatment of organic material for further processing to value-added products, comprising a loading section, a high-pressure retention section, a pressure relief section and a discharge section, wherein the said loading section serves to periodically load the high-pressure retention section while the high-pressure is being continuously maintained in the latter and the pressure relief section serves to periodically receive material from the high-pressure retention section and effectuate steam explosion of said material through pressure relief while continuously maintaining the pressure of the high-pressure retention section and the receiving section serves to receives material from the pressure relief section.

Another aspect of the invention relates to said loading section and/or said pressure relief section comprising a rotary dosing valve and more preferably a serial combination of a rotary dosing valve and a rotary discharge valve.

Another aspect of the invention relates to said loading section and/or said pressure relief section each (or either one) comprising a separate chamber, confined by two high-pressure valves, preferably floating ball vales or other suitable valves or combination of valves.

Another aspect of this invention relates to said pressure relief section being comprised of a separate chamber confined by three high-pressure valves, wherein said pressure relief section is fed from the side from the high-pressure retention section through a high-pressure valve, separating the high-pressure retention section from the pressure relief section. In these embodiments, pressure relief is upwards through a pressure relief valve placed above the feed port leading to a relief conduit extending from said pressure relief valve for receiving escaping steam and gas, preferably connected with a condenser for heat recovery. The substrate is released to the discharge section after the pressure relief through an exit valve at the bottom of the chamber.

This embodiment is advantageous, as solid material does not transfer through the relief valve in the explosion step, thus considerably reducing the potential for sealing problems due to abrasion or solid particles. Further protection of the relief valve from material splattering in the pressure relief process may be achieved through a protective mesh or meshes or protective collar/collars placed below the relief valve. This embodiment is also advantageous as instrumental erosion caused by high velocity solid material in the pressure relief section is avoided.
wherein the high-pressure retention section and loading sections can suitably be as described above, and the pressure relief section is as described above, comprising a chamber having a pressure relief valve connected to an upper portion of said chamber and a relief conduit extending from said pressure relief valve for receiving escaping steam, the chamber configured to operate at high pressure and to allow a sudden relief of pressure through the pressure relief valve, and having an exit port with a discharge valve for discharge of substrate after pressure release.

Another aspect of this invention relates to the use of pressurized gas, preferably air, to reach the desired steam explosion pressure prior to release into the pressure relief section. In this embodiment the desired temperature is reached through high-pressure steam injection, but for more economic operation the final release and/or processing pressure is reached by additional injection of pressurized gas, preferably air.

Another aspect of this invention relates to the use of pressurized gas, preferably air, mixed with CO₂ and preferably to a high partial pressure with CO₂. In this embodiment the CO₂ dissolves in the steam explosion media in the high-pressure zone and penetrates the subject material, further promoting its structural disintegration in the pressure release step of the steam- explosion process. Furthermore, the acidic nature of dissolved CO₂ promotes hydrolysis during the steam explosion process. Especially, where CO₂ penetrates material such as wood, acid-promoted hydrolysis of the cellulosic fraction of the internal of this material lessens its structural integrity. This makes such material more vulnerable in the steam explosion process, promoting its disintegration, and thus making it more accessible for further processing.

Another aspect of this invention relates to a combination of the steam explosion and hydrolysis equipment with a scrubber in which the steam exploding material is brought into contact with a CO₂-rich gas stream in a counter-flow configuration in the discharge section of the steam explosion equipment. In this embodiment the steam explosion and hydrolysis are conducted in an alkaline media, which serves in the discharge section as adsorbent for CO₂ and other acidic components contained in a counter-flow gas stream. These components may include H₂S, SOx and NOx, but are not limited to these.

The embodiments comprising carbon dioxide scrubbing are especially advantageous, where the pre-treated organic material is subsequently to be submitted to anaerobic digestion for the production of methane. Here the scrubber serves for removal of the CO₂ component and other acidic components from the CO₂ rich methane gas produced in this process. The integrated scrubber may also serve for binding of CO₂ where the pre-treated organic material is fermented for the production of ethanol or in other processes where removal of CO₂ and other acidic gases is required or advantageous.

The present invention further provides a process for pre-treating solid organic material with the above-described process steps and equipment. Accordingly, in one aspect, the invention provides a process for treatment of solid organic material that involves steam explosive disruption and making said material more accessible for further downstream processing, the process comprising the steps of:
a) receiving a material stream comprising solid organic material,
b) introducing the stream into a wetting and mixing section and wetting and mixing the solid organic material,
c) transferring the stream from said wetting and mixing section through a loading valve of a loading section into a loading compartment,
d) increasing the pressure in said loading compartment by introducing therein steam or pressurised gas such as pressurised air,
e) releasing material from said loading compartment to a high-pressure retention section,
f) feeding the material continuously through the high-pressure retention section while subjecting to a high temperature in the range of 170 to 250°C and a high pressure in the range of 10 to 40 bar,
g) transferring a material dose that has been fed through said high-pressure retention section through a valve to a discharge compartment, releasing pressure from said discharge compartment to attain a steam explosion effect on said material dose,
h) discharging said material dose into a lower pressure discharge section.

Various embodiments of the inventive process are described in further detail in the below detailed description.

### BRIEF DESCRIPTION OF FIGURES

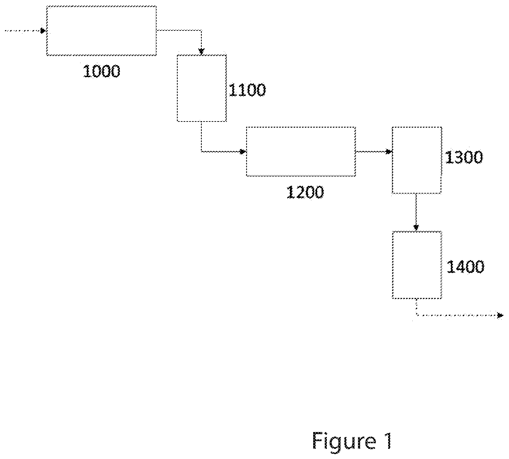

Figure 1a shows a schematic flow diagram of the steam explosion apparatus. Specifically, this embodiment comprises a wetting and conditioning section (1000), a loading section (1100), a high-pressure retention section (1200), a pressure relief section (1300). A discharge section (1400) is also shown, which under alkaline conditions can be configured as a scrubber for the removal CO₂ or other acidic components from a gas stream.
Figure 1b shows a corresponding diagram of the embodiment where explosive pressure relief is attained through a pressure relief valve, releasing pressure above the substrate surface and releasing escaping steam and gas through a conduit.

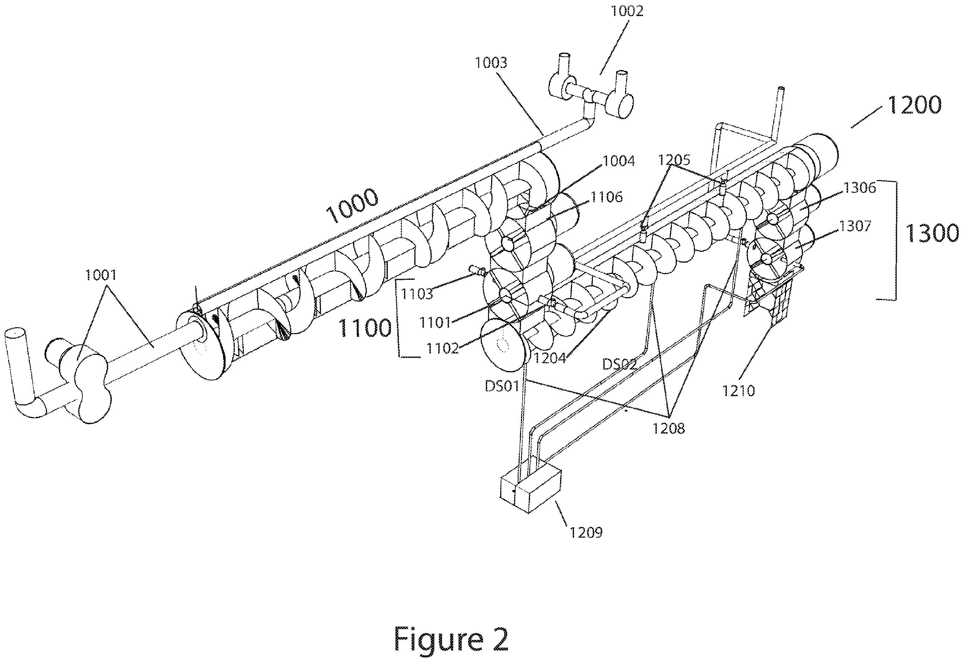

Figure 2. shows an explosive perspective illustration of one embodiment of the steam explosion reactor, specifically the wetting and mixing section, the loading section, the high-pressure retention section and the pressure relief section. In this embodiment the loading section and the pressure relief section are realized each, by two rotary valves in tandem and the high-pressure retention section comprises a screw conveyor. The discharge section is not shown in this figure.

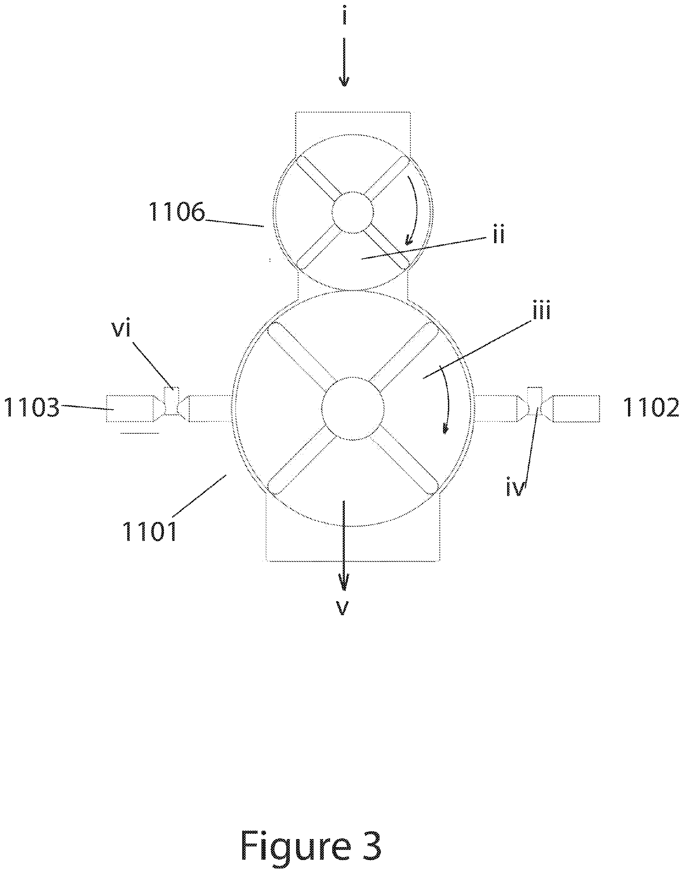

Figure 3. Schematic representation of the dosing and loading process in the loading section, using the combination of two rotary valves in tandem. Individual compartments and their positions are assigned to the respective steps in the dosing/loading process and the individual steps are numbered subsequently in the order of their execution.

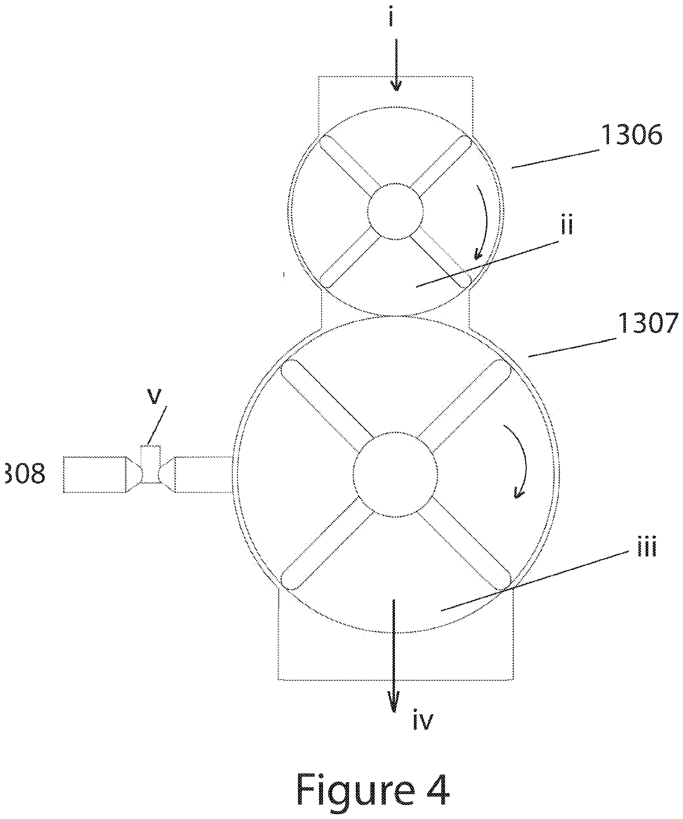

Figure 4. Schematic representation of the pressure relief and discharge process from the pressure relief section, using the combination of two rotation valves in tandem. Individual compartments and their positions are assigned to the respective steps in the process and the individual steps are numbered subsequently in the order of their execution.

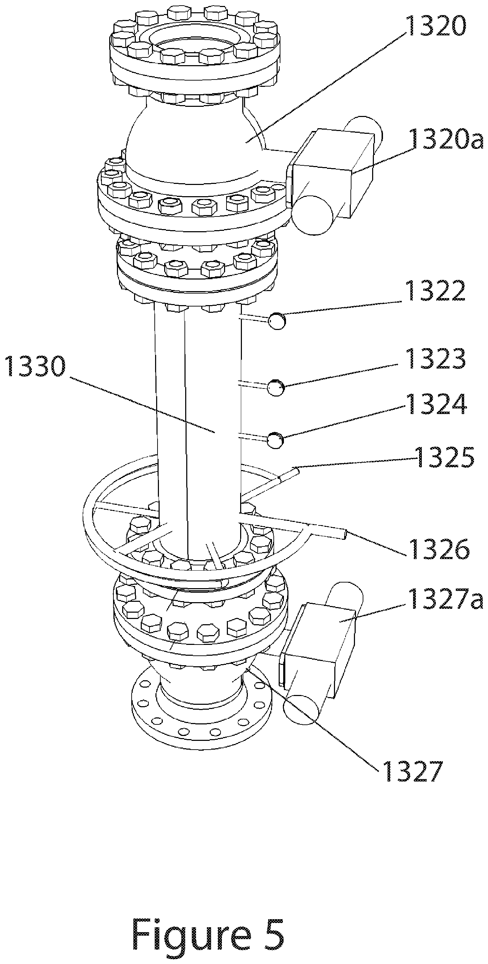

Figure 5. Perspective view of an example of the loading and the pressure relief section, where these sections are configured with a chamber 1330 confined by high-pressure loading **(1320)** and discharge **(1327)** valves. Also shown are injection ports for steam and pressurized air. In this example the loading and the pressure relief section are preferably configured in the same principal manner and are thus, in this embodiment the example shown in figure 5 applies equally to both these sections. However, the loading section chamber is between the wetting and condition section and the high-pressure retention section, while the pressure relief chamber is between the high-pressure retention section and the discharge section. In this example, the valves confining the chambers are shown as high-pressure ball valves, preferably these are floating-ball valves.

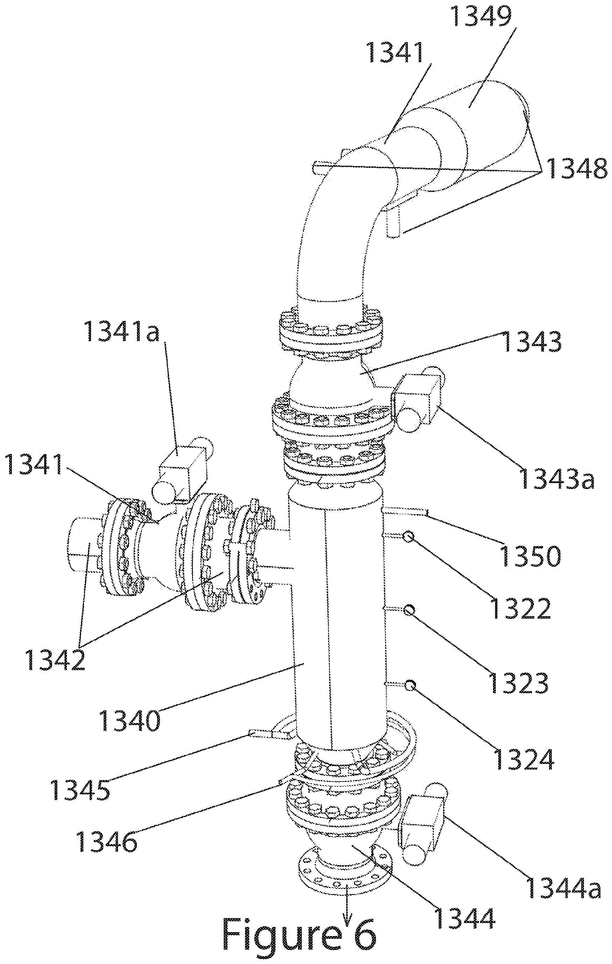

Figure 6. Perspective illustration of the pressure relief section of the steam explosion unit in an embodiment where the pressure relief section is fed through a side inlet (1341, 1342), from the high-pressure retention section. Pressure relief is realized upwards, through a pressure relief valve (1343) placed above the substrate material and the condensed steam, and above the inlet at the side of the chamber. In this embodiment the steam explosion material does not protrude or splatter beyond the pressure relief valve (1343), which may additionally be protected by a splash guard such as a downward pointing rim placed in a collar on the inner side of the compartment, between the feed port and the pressure release valve or through a mesh or meshes (not shown). Discharge of substrate from the pressure relief section is realized through a discharge valve (1344) at the bottom of the chamber.

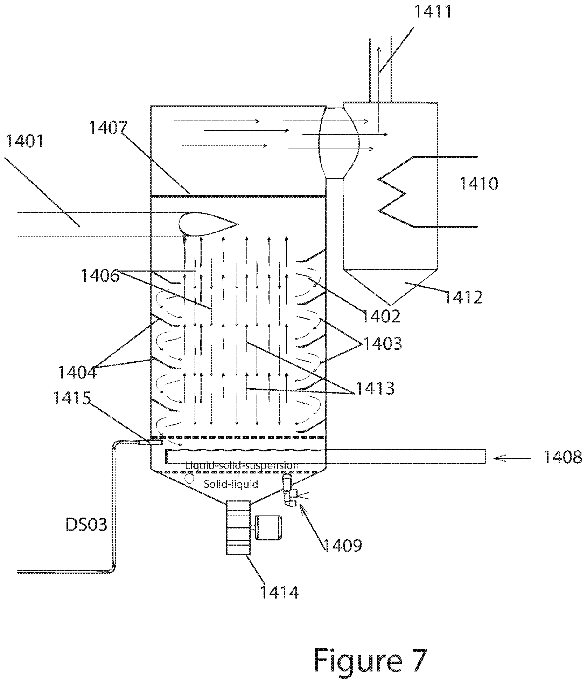

Figure 7. Schematic illustration of a version of the discharge section where this section is configured to act as a carbon dioxide scrubbing unit at the same time. This enables, where advantageous, economic scrubbing of CO₂ or other acidic components from gases when the steam explosion process is run under alkaline conditions.

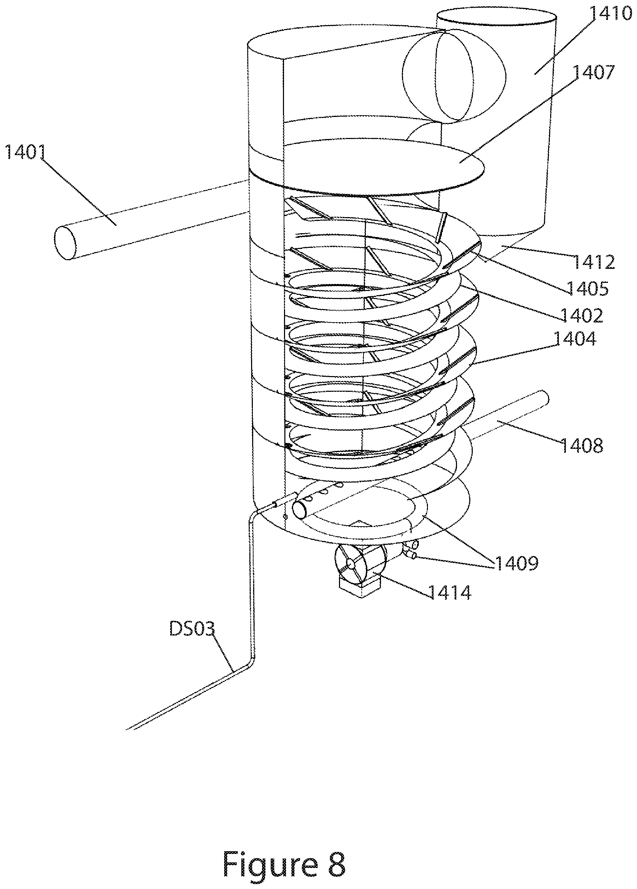

Figure 8. Perspective exploded view of a version of the discharge section, where this section is configured to act as a carbon dioxide scrubbing unit at the same time.

### DETAILED DESCRIPTION

In the following, exemplary embodiments of the invention will be described, referring to the figures. These examples are provided to provide further understanding of the invention, without limiting its scope.

In the following description, a series of steps are described. The skilled person will appreciate that unless required by the context, the order of steps is not critical for the resulting configuration and its effect. Further, it will be apparent to the skilled person that irrespective of the order of steps, the presence or absence of time delay between steps, can be present between some or all of the described steps.

In an embodiment of the invention, the continuous-flow or semi-continuous-flow steam explosion system comprises an upstream grinding and homogenizing section, an upstream wetting and mixing section **(1000)** also referred to as conditioningsection (or specifically as steam-explosion-conditioning section), a loading section (1100), a high-pressure retention section **(1200)** (also referred to as high-pressure-heating-retention section or high-pressure high-temperature retention section) and a pressure relief section (1300). A discharge section (1400) for receiving the material from the pressure relief section may optionally form part of the apparatus or can be seen as representing any suitable receiving unit for further processing of the processed substrate stream.

Where steam explosion is conducted under alkaline conditions the discharge section can be suitably configured as a CO₂ scrubbing section **(1400).**

The discharge section of the steam explosion and CO₂ scrubbing unit, when run under alkaline condition, can serve at the same time as absorber for CO₂ or for other acidic components such as but not limited to H₂S, SOx and NOx from a gas stream. In this context scrubbing/scrubber refers to the removal of acidic gas components from a subject gas through acid-base reactions of said gas with the expanding alkaline substrate subjected to the steam explosion. Equations 1a, b and c show the respective reactions for the example of CO₂ scrubbing using sodium hydroxide as the alkaline medium.

CO₂ (g) + H₂O ⇄ H₂CO₃ (aq) 1a

H₂CO₃ (aq) + NaOH (aq) # NaHCO₃ (aq) 1b

NaHCO₃ (aq) + NaOH (aq) # Na₂CO₃ (aq) 1c

An example where this is advantageous is the removal of CO₂ and other acidic gases from CO₂ rich methane gas from anaerobic digestion, from for example the pre-treated material from said steam explosion. Other examples are the binding of the CO₂ from fermentation of the pre-treated material from the steam explosion and the binding of CO₂ and other acidic components of the flue gas from a steam boiler providing steam for the apparatus/process of the invention.

In general, in the steam explosion process described herein, the organic material stream has preferably been shredded within an upstream grinding and homogenisation section. The material is then fed into the far end of a wetting and mixing section **(1000)** and conveyed under mixing towards its exit point. During this step the solid substrate is wetted to achieve the desired water content optimal for the steam explosion process and at the same time pH adjustment is achieved.

The wetting and mixing section **(1000)** preferably constitutes a actively fed **(1001)** conveying mixer equipped with provided with a liquid mixing and inlet system (**1002,** preferably connected to an armature in the mixing conveyor section though a top-down spraying configuration **(1003).**

For alkaline steam explosion integrated with CO₂ scrubbing, as described in more detail here below, the aqueous solution is alkaline, preferably through solution of sodium or potassium hydroxide added to the wetting water in a liquid/liquid mixer.

The steam explosion unit may, however, also be driven with pH neutral or acidic feed, with non-pH adjusted feed or with feed mixed with any other additives suitable for promoting the intended processes.

In one embodiment, the exit port of the wetting and mixing section is connected vertically to the high-pressure section via a loading section comprising a rotationdosing valve **(1106)** and a high-pressure, rotating valve **(1101),** which may advantageously be in a positive displacement configuration. At the exit point of the wetting and mixing unit **(1004),** the substrate enters the rotating dosing valve **(1106),** which doses the substrate into an ambient pressure compartment of the high-pressure rotary valve **(1101).** The rotating dosing valve **(1106)** serves to avoid overload of the high-pressure rotary valve, which could cause unwanted strain on the high-pressure sealing. After loading at ambient pressure, the high-pressure rotation valve rotates to a sealed position where the substrate holding valve chamber is brought up to the desired pressure (preferably in the range 10-30 bar, typically about 1 bar above that of the high-pressure retention section) and temperature, through steam injection (typically 180-250°C) **(1102).** The high-pressure rotating valve then rotates further to a vertical placement above the entrance port of the high-pressure retention section. This process is shown schematically in **Fig. 3****,** where individual compartments (their positions) are assigned to individual steps and the individual steps are indicated with roman numbers **i-vi** in the order of their execution, as follows:
i. Substrate exits the wetting and conditioning section and fills compartment of rotary dosing valve **(1106);**
ii. Rotary dosing valve rotates to deliver the substrate to a compartment of the high-pressure rotary valve **(1101);**
iii. High-pressure rotary valve rotates to seal off the dosing valve;
iv. The substrate in the high-pressure rotary valve is brought to the desired high-pressure and high temperature conditions of the high-pressure retention section by means of steam injection and eventually additional gas (air) injection **(1102);**
v. High-pressure valve rotates to deliver substrate to the high-pressure retention section;
vi. High-pressure valve rotates to pressure relief position where pressure is relieved **(1103)** to equal the atmospheric pressure of the delivering conditioning and vetting section, through pressure relief.

Assisted by the differential pressure and gravitation the now pressurized chamber of the high-pressure rotation valve is discharged into the high-pressure retention section **(1200)** of the steam explosion unit before rotating further into a second sealed position where pressure is released (through pressure release **1103**).

Pressure strain on the rotation-valves may be reduced by using rotation-valves with more compartments and gradually increasing the pressure in individual compartments, lowering the differential pressure between individual compartments.

In a positive displacement configuration applied in some embodiments, pressurization and depressurization is assisted by the respective chamber volume variation through the rotary cycle.

High-pressure steam for the process, is typically provided by a boiler. Where the pre-treated material is to be further processed for fuel production, part of the produced fuel may be used to drive the boiler. This may for example be pure methane or any methane/CO₂ mixture, from an anaerobic digestion unit, biodiesel or any mixture of fats and oils where these are separated from the steam-exploded material and suitably processed.

In the high-pressure retention section **(1200),** the loaded substrate is conveyed from the loading point to the exit point by means of an adjustable speed conveyor **(1204)** or mixing conveyor, allowing the retention time to be continuously adjustable over a wide time range.

The high-pressure retention section is equipped with steam injection ports **(1205)** to achieve the appropriate pressure and temperature and for its maintenance by make-up injections. Two such injection points are shown as examples in **Fig. 2****.**

In one embodiment, the exit port of the high-pressure retention section is connected vertically to a rotating dosing valve **(1306)** and a high-pressure rotary relief valve **(1307).** At the exit point, the substrate is conveyed by the rotating dosing valve **(1306)** to a compartment of the high-pressure rotary relief valve **(1307),** which at that timepoint faces the exit port of the high-pressure retention section).

The high-pressure rotary valve **(1307)** then rotates to open the pressurized compartment to ambient pressure, enabling the steam explosion, before rotating to a re-pressurization position, this position being equipped with a steam injection port. From this position the compartment rotates back to the fill position. This process is shown schematically in **Fig. 4****,** where individual compartments (their positions) are assigned to individual steps and the individual steps are numbered subsequently with roman numbers i-v in the order of their execution, as follows:
i. Substrate exits high-pressure retention section and fills compartment of rotary dosing valve **(1306);**
ii. Rotary dosing valve rotates to seal off the exit of the high-pressure retention section and to deliver the substrate to a compartment of the high-pressure rotary valve **(1307);**
iii. High-pressure rotary valve rotates to deliver substrate;
iv. Substrate is discharged under pressure relief, where the sudden drop in pressure causes steam explosion;
v. Valve rotates to pressure adjustment position and pressure is increased via steam and/or gas (air) injection **(1308).**

In this configuration, heat efficiency is maximized by dynamic synchronization of the steam injection into the respective compartments of the high-pressure rotary loading valve, the high-pressure retention section and the re-pressurization section of the discharge valve. All injections are controlled individually in response to variations in pressure and temperature caused at the relief and entrance side and through heat loss. Pressure and temperature are preferably monitored continuously along the high-pressure retention section supplying individual data along the section. This data **(DS01)** is feed to the PIT processing unit **(1209)** supplying control signal **(DS02)** to the steam injection ports of the high-pressure retention section **(1205)**.and to steam injection ports of the loading **(1102)** and pressure relief section **(1308).** These are synchronized to this signal along the high-pressure retention section to maintain close to constant conditions. Additional signal is supplied to adjust the speed of the rotating conveyor **(1204).** Three PIT monitoring points are shown as examples in **Fig. 2** **(1208).**

In other embodiments one or both of the loading section and the pressure relief section of the steam explosion unit can constitute a separate chamber **(1330),** separated from the conditioning section and the high-pressure retention section and the high-pressure retention section and the discharge section, respectively, by valves such as but not limited to high-pressure ball valves, knife gate valves or slide gate valves, preferably a floating-ball valve, or other suitable high-pressure valves. An example is shown in figure 5, which is applicable to both a loading chamber and pressure relief chamber. In both cases (loading chamber and pressure relief chamber) the chamber is provided with a high-pressure loading valve **(1320)** and a high-pressure discharge valve **(1327)** both equipped with appropriate actuators **(1320a** and **1327a,** respectively). The chamber is equipped with injection ports for steam **(1325)** and/or gas (such as air) **(1326)** and with pressure **(1323),** temperature **(1324)** and level **(1322)** sensors.

In the above described embodiments having a loading chamber as described, the loading chamber is loaded from the top, receiving material from the conditioning section under ambient pressure conditions. During dosing, the loading chamber is separated from the high-pressure retention section by a high-pressure discharge valve **(1327)** in closed position. The dosing valve **(1320)** is then moved into closed position, in response to readings from the level sensor **(1322),** before the loading chamber is pressurized and brought to the desired temperature through steam and optional gas injection **(1325** and **1326,** respectively). Generally, the loading chamber is in this step brought to a pressure slightly above that of the high-pressure retention section, typically 1 bar higher. Assisted by the differential pressure and gravitation the now pressurized loading chamber is discharged into the high-pressure retention section **(1200)** of the steam explosion unit by opening the discharge valve of said chamber **(1327).** The loading valve is then brought into closed position and the loading chamber is depressurized with a pressure relief mechanism before the next dose is loaded. In this configuration the steam explosion step is achieved similarly by loading the pressure relief chamber with its loading valve **(1320)** in open position and relief/discharge valve **(1327)** in closed position. The loading valve **(1320)** is then brought into closed position, in response to readings from the level sensor **(1322),** and the steam explosion is effectuated through opening its relief/discharge valve **(1327)** to the discharge section **(1400).** The relief/discharge valve is then closed and the pressure relief chamber is brought to the same pressure or pressure slightly below that of the high-pressure retention section before its loading valve is opened again for reloading.

In the above embodiments heat efficiency is maximized by dynamic synchronization of the steam injection into the loading chamber **(1330),** the high-pressure retention section **(1200)** and the pressure relief compartment **(1330).** This is done in response to variations in pressure and temperature caused at the relief and entrance side and through heat loss. Pressure and temperature are preferably monitored continuously along the high-pressure retention section **(1200)** and from the respective chambers **(1330).** This data is fed to a PIT processing unit **(1209)** supplying control signal to the respective steam injection ports. These are synchronized based on the signals along the high-pressure retention section to maintain substantially constant conditions. Additional signal is supplied to adjust the loading and discharge frequency from the respective chambers and the speed of the conveyor **(1204)** transferring material through the high-pressure retention section.

Loading and pressure relief may also be realized with any combination of rotary valves or separate chambers as described above, at the loading and discharge sides of the high-pressure retention section, respectively. Where advantageous the loading section and the high-pressure retention section may also be run at reduced pressure compared to the pressure relief section and the pressure of the pressure relief section may be boosted up by steam injection and/or gas (air) injection prior to discharge.

Independent of the preferred loading and discharge mechanisms chosen, in one embodiment the steam explosion may be assisted by suitable injection of pressurized air. In this embodiment the desired temperature is achieved through steam injection as described above, but the final pressure is realized through additional injection of pressurized air. In this configuration, pressurized air injection ports are preferably provided along with the steam injection ports provided for the loading section, the high-pressure retention section and the pressure relief section of the steam explosion unit. These are shown in the exemplary embodiments of the loading and pressure relief sections in Figs. 3, 4 and 5, but may also apply where these sections are realized by any other suitable means.

In one embodiment shown in a perspective illustration in figure 6, the pressure relief section comprises a separate chamber **(1340)** confined by three high-pressure valves: a loading valve **(1341)** connecting a feeding port **(1342)** at the side of the pressure relief chamber to the exit of the high-pressure retention section **(1200),** a pressure relief valve **(1343)** placed above the feeding port and a discharge valve **(1344)** at the bottom of the pressure relief chamber. All three high-pressure valves are equipped with appropriate actuators **(1341a, 1343a and 1344a,** respectively). The conveying of the substrate from the high-pressure retention section to the pressure relief chamber in this embodiment, may be realized through the main conveyer in the high-pressure retention section and/or through an intermediate pump placed between the high-pressure retention section and the pressure relief section. Pressure adjustment may be realized through steam **(1345)** and/or pressurized air **(1346)** injection into the pressure relief chamber after loading and closing of the respective loading valve **(1341).** Steam explosion is effectuated, in this embodiment, through pressure relief through the pressure relief valve **(1343)** placed above the feeding port **(1342).** The pressure relief valve may be protected by a splash guard, such as a conical downwards-tilted collar and/or a protective mesh placed between the loading port and the relief valve (not shown) reducing splashing of solid and liquid material in the steam explosion process reaching critical components of the pressure relief valve. Gas and steam release through the pressure relief expands through an upper portion of said chamber and a relief conduit extending from said pressure relief valve for receiving and transmitting escaping steam and/or gas, the conduit comprising a heat exchanging condenser **(1347),** discharge ports **(1348)** and optional silencer **(1349).** The condenser serves for removal of the steam and heat recovery from condensation of the steam and from the hot gas. After the pressure relief the steam exploded material is discharged though a discharge valve **(1344)** at the bottom of the pressure relief chamber for further processing to value-added products. To accelerate discharge of the steam-exploded material the discharge may be assisted with pressurized air injected above the substrate material **(1350)** with the pressure relief valve placed in closed position. After discharge the discharge valve is closed and the pressure relief chamber is repressurized prior to re-loading.

Advantageously, the loading of the high-pressure retention section and the pressure relief compartment, the pressure relief and the material discharge from said compartment is synchronized such that pressure drop in the high-pressure retention section is minimal during operation. Retention time in the high-pressure retention section is adjustable over a wide range, by adjustment of the conveying speed, which again is synchronized with the loading and relief mechanism. The synchronization is advantageously controlled via a control unit such as a PLC system receiving readings from temperature **(1324)** and pressure **(1323)** and level **(1322)** sensors installed in the pressure release chamber and controlling the convening speed and timing sequence of loading, pressure relief and discharging as well as steam and gas injections, as described in illustrative non-limiting examples for other embodiments herein above.

Where advantageous, pressure boosting through pressurized air injection may also be confined to the pressure relief section boosting up the pressure of this section prior to its discharge while the loading and high-pressure retention sections are run at comparatively lower pressure.

In another embodiment, where pressure boosting is achieved through pressurized air injection said pressurized air may preferably be air mixed to a high partial pressure with CO₂. In this embodiment the injection of the CO₂ enriched pressurized air is injected below the surface of the substrate and condensed steam in the respective sections, preferably from their bottom (1325). The CO₂ dissolves in the steam explosion media in the high-pressure zone(s) as the corresponding carbonic acid (Eq 1a), providing acidic conditions as shown in equations 2a and 2b:

H₂CO₃ (aq) + H₂O # HCO₃⁻ (aq) + H₃O⁺ (aq) 2a

HCO₃⁻ (aq) + H₂O # CO₃²⁻ (aq) + H₃O⁺ (aq) 2b

The CO₂ penetrates the subject material, further promoting its structural disintegration in the pressure release step of the steam- explosion process in accordance to the reversed reaction as shown in Eq. 1a. The acidic nature of dissolved CO₂, shown in Eq. 2a and 2b, further promotes hydrolysis during the steam explosion process. Especially, where CO₂ penetrates material such as wood, such acid promoted hydrolysis of the cellulosic fraction of the internal of this material lessens its structural integrity. This makes such material more vulnerable in the steam explosion process, further promoting its disintegration, and making it better accessibility for further processing. The partial pressure of CO₂ can suitable be chosen depending on the pressure and temperature ranges applied, such that the partial pressure provides an effective concentration of CO₂ while being safely below the vaporsublimation/deposition phase boundary.

In some embodiments, where discharge is under high-pressure and constitutes the steam explosion step in the process, it is found advantageous that the discharge port of the pressure relief section is partially obstructed with suitable constructions to utilize the mechanical force in the steam explosion for further mechanical surface roughening/shredding of the extruding material. This is exampled in **Fig 2** by a shredder teeth arrangement **(1210)** intended for additional surface roughening of wood chips.

The steam explosion equipment will generally be operated at a pressure in the range from about 10 bar (1.000 kPa) or from about 12 or from about 14 or from about 15 or from about 16, or from about 18 or from about 20 bar, to about 40 bar, or to about 38 bar, or to about 36 bar, or to about 34 bar, or to about 32 bar, such as to about 30 bar, such as to about 28, or to about 27 or to about 26 or to about 25 or to about 24 bar.

The temperature in the steam explosion process is typically in the range of about 170-250°C such as the range 180-250°C, the selected temperature will typically depend on the desired pressure, meaning that the temperature and pressure are maintained in a relationship so that the pressure is around or just above the vapour pressure of water. For example, at a temperature of 180°C, the vapour saturation pressure of water is just over 10 bar, at 200°C the saturation vapour pressure is about 15,5 bar, at 220°C is about 23,2 bar, and at 250°C the vapour saturation pressure is about 40 bar.

In some embodiments the steam explosion reactor is operated at a temperature in the mentioned range and at a pressure corresponding to or close to the vapour saturation pressure of water at the respective temperature. Accordingly, in some embodiments, the steam explosion reactor is operated at a temperature in the range of about 180-200°C and a pressure in the range of about 10-16 bar, in some embodiments the steam explosion reactor is operated at a temperature in the range of about 200-220°C and a pressure in the range of about 15-23 bar, or in the range of about 220-240°C and at pressure in the range of about 23-33 bar.

Where the steam explosion is conducted under alkaline conditions and applies a pressure relief section releasing pressure with the substrate to the discharge section, said discharge section can at the same time constitute a CO₂ scrubbing unit. In this configuration the upper part of the CO₂ scrubber **(1400)** is in a cyclone type configuration, designed to promote perpendicular dispersion towards the centre of the scrubber for optimal contact of the alkaline material with a counter-flow of CO₂ rich or other acidic gas containing exhaust.

As understood from herein, in preferred embodiments the carbon dioxide scrubbing unit constitutes an integral part of the continuous-flow steam explosion reactor when the steam explosion is operated under alkaline conditions. An upper part of the carbon dioxide scrubbing unit is preferably adapted to provide delivery of a stream of material from the high-pressure retention section of the steam explosion unit, such that flow of material provided through the pressure relief is directed to a cyclone-like pattern within the carbon dioxide scrubbing section, but at the same time splashing and dispersion towards the centre of the scrubbing section is promoted. Hence, the dispersion of the alkaline, aqueous material stream (the absorber) in the region where it meets the carbon dioxide rich gas stream serves to enhance scrubbing efficiency.

The terms "carbon dioxide scrubber" and "carbon dioxide scrubbing" as used herein refer to that carbon dioxide is directed/injected into the scrubber to be adsorbed, thus the scrubber serves as regular carbon dioxide scrubber which has the general function of removing or reducing CO₂ content from a CO₂ rich stream.

Where the discharge section functions as a CO₂ scrubbing unit, the loaded compartment of the high-pressure rotary exit valve or the pressure relief chamber is preferably discharged under an appropriate angle through an inlet tube **(1401)** at the upper part of the combined discharge and CO₂ scrubbing section **(1400),** directing the high-speed steam/substrate downwards on to a cyclone type spiral configuration **(1402)** with clearance from the inner walls of the primary scrubber compartment. The horizontal/angled part of the discharge tube **(1401)** is separated from the gas void section below the gas exhaust port with a demistifier mesh **(1407).**

The term cyclone pattern as used herein refers to general circular or spiral pattern. The entrance angle of the waste stream and spiral-shaped guides inside the scrubber enhance the cyclone-like flow. The entrance angle is in some embodiments in the range of about 5° to 30° from horizontal, that is, downwardly tilted from horizontal, such as at angle in the range from about 5° or from about 10°, to about 30° or to about 25° or to about 20°.

The spiral-shaped guides inside the scrubber, however, are designed to partly disrupt the cyclone-like flow, causing effective splashing and dispersion of the incoming stream towards the centre region of the scrubbing section and maximize contact with the carbon dioxide rich gas stream. In some embodiments the scrubbing unit comprises at least one spiral-shaped insert and preferably at least two. In one embodiments the unit comprises two internal spirals vertically offset with respect to each other, with the upper spiral having clearance from the inner wall of the scrubber while the lower spiral has no clearance. Such exemplary configuration is shown in **Figs 7** and **8****.** Through the high velocity of the discharging material the heavier fraction and condensed steam is forced towards the inner wall of the scrubber/cyclone confinement with a downward velocity component provided by the initial angle of entrance. This fraction is caught by a corrugated spiral-plate **(1404)** below and parallel to the cyclone spiral with wall clearance **(1402).** The corrugation **(1405),** the downward slope of the spiral plate **(1404)** and the downwards velocity of the substrate/condensate **(1403)** partly directs it along the surface of the lower spiral plate **(1404)** towards the centre where it is dispersed by an upwards directed protrusion terminating the inner side of the spiral plate, thus enhancing the contact of the alkaline suspension with the counter flow of CO₂ rising up through the scrubber. The dispersing substrate/condensate **(1406)** falls down the centre of the scrubber/cyclone confinement, along with steam condensing in the central region and at the demistifier **(1407),** to accumulate in the settlement region of the scrubber/cyclone.

The horizontal/angled part of the discharge tube **(1401)** is separated from the gas void section below the gas exhaust port with a demistifier mesh **(1407).**

Carbon dioxide flows into the carbon dioxide scrubbing unit through at least one carbon dioxide inlet **(1408, 1409)** which is preferably provided within a lower part of the unit, thus bubbles through the alkaline material accumulation in the settlement region at the bottom of the scrubbing section before rising through the scrubbing section and meeting the stream of dispersed alkaline material moving downwards within the scrubbing section.

The CO₂ containing gas stream or gas stream containing other acidic gases is preferably fed from the bottom through a micro-bubble dispenser **(1409).** This is preferably through aspirators **(1409)** in a tilted, lateral arrangement, driven by circulation of the low solid aqueous suspension taken from the upper part of the suspension section. In this arrangement the aspirators serve at the same time for agitation of the liquid solid suspension, achieving consistent composition at the exit port of the section and avoiding clogging. Alternatively, CO₂ containing exhaust is fed through a micro-bubble or other, passive, dispersion set-up **(1409, 1408).**

The CO₂ containing gas stream protrudes through the substrate settlement region and rises up the centre of the scrubber **(1413)** where it is further in contact with the alkaline adsorbing suspension in the upper, central part of the condensation section of the scrubber before exiting with the dry fraction of the steam through a condenser **(1410)** at the gas exit port of the condenser **(1411).**

The remaining steam is removed in the condenser **(1410)** with the CO₂ lean gas (CH₄ for example, when scrubbing CO₂/CH₄) exiting the gas exit port **(1411)** and the condensate exiting the drain port **(1412).** The heat of condensation is for example used to preheat the aqueous fed for the wetting unit.

The settlement region of the combined pressure relief and CO₂ scrubbing section **(1400)** may further be equipped with a cooling spiral or other heat exchange elements to partially recapture the heat from the steam explosion, thus lowering the substrate temperature and preheating for example the water input for the steam generation boiler providing the system. Such cooling elements may also be installed or extend to the upper section of the scrubbing unit and are preferably in contact with the guiding spirals to effectuate rapid cooling of the substrate already in the upper section of the scrubber.

The scrubber is periodically emptied trough a rotary valve or any other suable valve **(1414)** at the bottom of the settlement region, preferably synchronized with the pressure relief section feeding the scrubber with substrate material through a signal (DS03) liquid level sensor **(1415)** suitably arranged in the scrubber.

Where advantageous, the gas exit port **(1411)** leads to a secondary scrubber that may be operated under elevated pressure and at lowered temperature. The secondary scrubber is fed with the preferably cooled liquid fraction of the primary absorber/scrubber in a conventional spray configuration from the top and with the CO₂ lean exhaust from the primary scrubber from the bottom.

The carbon dioxide scrubbing unit, where the steam explosion is conducted under alkaline conditions is operated at a pressure in a range from about 1 bar, such as from about 1,2 bar, such as from about 1,5 bar, such as from about 2 bar, to about 5 bar, or to about 4 bar, and alternatively provided with cooling elements for lowering the substrate temperature and for recapture of heat from the steam explosion unit.

The scrubbing functionality of the steam explosion unit advantageously serves to remove CO₂ and other acidic gases from for example a raw CO₂/CH₄ mixture produced from an anaerobic digestion process of the pre-treated steam-exploited material or the CO₂ produced in a fermentation process of this material and/or to remove CO₂ and other acidic gases from the flue gas from the steam boiler providing the system.

Further, in the process the CO₂ serves to lower pH and buffer the alkaline substrate/aqueous-extract (the absorber) generated in the steam explosion process, before its further processing to value added products. Where the extract provided in the steam explosion process is intended for anaerobic digestion for the production of methane, the increased carbonate and bicarbonate concentration provided through the CO₂ adsorption, also promotes of the methane production.

The liquid-solid suspension at the bottom of the relief and CO₂ scrubbing section is periodically released as fractions through a discharge valve **(1413).** These fractions are subjected to conditioning and separation as appropriate prior to further processing to value added products. The discharge of the discharge and CO₂ scrubbing section is preferably synchronized with the discharge from the steam explosion unit to achieve continuous load conditions in the process.

A secondary scrubber may be installed with conventional adsorbed material or such that it is fed with the pre-filtered liquid fraction accumulating in the settlement region of the primary scrubber and may be in a conventional top-down spraying configuration, at elevated pressure and reduced temperature with the CO₂ lean exhorts from the primary scrubber fed from the bottom. Accordingly, a secondary scrubber in the system may in some embodiments be operated at a pressure in the range of about 5 to 150 bar, such as at a pressure value in the range from about 5 or from about 10 or from 15 or from about 20 or from about 30 or from about 40, to about 150 or to about 140 or to about 130 or to about 120 or to about 110 or to about 100 or to about 90 or to about 80 or to about 70 or to about 60 or to about 50 bar, and the temperature is preferably in the range 5 to 40°C such as in a range from about 5°C or from about 10°C or from 1 about 5°C or from about 20°C, to about 50°C or to about 45°C or to about 40°C or to about 35°C or to about 30°C.

## Claims

1. A continuous-flow steam explosion reactor for pre-treatment of organic material for further processing to value-added products, comprising:
a loading section (1100),
a high-pressure retention section (1200),
a pressure relief section (1300), and
a-discharge section (1400),
and wherein said high-pressure retention section (1200) comprises at least one adjustable-speed conveyor (1204) for transporting a stream of source material through said section, further comprising steam provision means connected to a source of steam, for providing steam into at least said high-pressure retention section (1200), wherein said loading section (1100) is configured to transfer material at ambient pressure upstream of the loading section (1100) to said high-pressure retention (1200) section while retaining high pressure and temperature in the high-pressure retention section (1200) by means of steam injection to said loading section (1100),
**characterised in that** said pressure relief section (1300) is configured to transfer material from said high-pressure retention section (1200) to a discharge section (1400) while retaining high pressure in the high-pressure retention section (1200) by means of steam injection to said pressure relief section (1300), and
wherein said steam injection means in said loading section (1100) and said steam injection means in said pressure relief section (1300) and loading and release from sections are synchronised such that pressure drop in the high-pressure retention section (1200) is minimal and high operating pressure and temperature is maintained during operation.

2. The continuous-flow steam explosion reactor according to claim 1, comprising gas provision means connected to a source of pressurized gas into at least one of said high-pressure retention section (1200), said loading section (1100) and said pressure relief section (1300), to generate at least part of high pressure through pressurized gas injections, wherein high temperature is achieved through steam injections.

3. The continuous-flow steam explosion reactor according to claim 1 or 2, wherein said loading section (1100) comprises at least one rotary dosing valve (1106) and at least one rotary discharge valve (1101), wherein said rotary dosing valve is dimensioned and configured to transfer a suitable dose of substrate to the rotary discharge valve (1101), and said loading section (1100) comprises pressure adjustment means coupled to said steam provision means and/or said gas provision means, to adjust the pressure of a compartment in said rotary discharge valve (1101) such that the pressure and temperature within said high-pressure retention section (1200) is substantially maintained when said compartment discharges material to said high-pressure retention section (1200).

4. The continuous-flow steam explosion reactor according to any of claims 1 to 3, wherein said pressure relief section (1300) comprises at least one rotary dosing valve (1306) and at least one rotary discharge valve (1307), wherein said rotary dosing valve is dimensioned and configured to transfer a suitable dose of substrate to the rotary discharge valve, and said pressure relief section comprises pressure adjustment means coupled to said steam provision means and/or said gas provision means, to adjust the pressure and temperature of a compartment in said rotary discharge valve after discharge, such that the pressure within said high-pressure retention section and said rotary dosing valve is substantially maintained when said compartment returns to a position for receiving material from said rotary dosing valve.

5. The continuous-flow steam explosion reactor according to any of claims 1 to 3, wherein said loading section (1100) and/or said pressure relief section (1300) respectively comprises at least one loading valve and at least one discharge valve, and at least one loading chamber in between said valves or being part of said discharge valve, said loading chamber is coupled to said steam injection ports and optional gas injection means and provided with pressure relief mechanism, to pressurize and de-pressurize said chamber before discharging and loading, respectively, and to bring to desired temperature prior to discharging.

6. The continuous-flow steam explosion reactor according to claim 4 or 5, wherein said pressure relief section (1300) comprises a pressure-relief valve (1343) connected to an upper portion of said chamber and wherein said loading valve of the pressure relief section is positioned below said pressure-relief valve, and a relief conduit extending from said pressure-relief valve for receiving escaping steam and/or gas, said discharge valve positioned at the bottom of the pressure relief section, wherein said relief conduit preferably comprises a condenser, a discharge port and optional silencer.

7. The continuous-flow steam explosion reactor according to any of claims 2-6, wherein said pressurized gas comprises at least for some section(s) a partial pressure of carbon dioxide.

8. The continuous-flow steam explosion reactor according to any of the preceding claims, which is configured for operating as an alkaline steam explosion reactor,
comprising at least one integrated scrubber unit, wherein said discharge section of the steam explosion reactor serves at the same time as said scrubber unit in that the section is configured such that discharge material from the steam explosion reactor serves as scrubbing medium, wherein the scrubber unit comprises at least one gas inlet provided within a lower part of the scrubber unit, such that when operated, an alkaline stream of material from the high-pressure retention section meets a gas stream to facilitate carbon dioxide scrubbing and/or scrubbing of other acidic gas components from said gas stream, optionally comprising a secondary scrubber which is configured to be fed with a liquid fraction from said scrubber unit as scrubbing media.

9. The continuous-flow steam explosion reactor according to claim 8, wherein an upper part of the scrubber unit is adapted to receive delivery of a stream of material from the pressure-relief section under an angle with respect to the central axis of the scrubber unit, to direct said stream of material into a cyclone patter by means of at least one internal spiral and preferably the scrubber unit comprises two inserted spirals which are vertically offset with respect to each other with the upper spiral having clearance from the inner wall of the scrubber while the lower spiral has no clearance, which effectuates partial velocity component perpendicular to the primary cyclone pattern flow of the stream of waste within the scrubbing unit, effectuated by material conveyed from the upper spiral to the lower spiral along the inner wall of the scrubber unit.

10. The continuous-flow steam explosion reactor according to claim 9 wherein said lower spiral is provided with a corrugated pattern partly guiding material towards the center of the scrubber and a protruding rim at the inner edge of the spiral causing the perpendicular component of the material to splash and disperse towards the center of the scrubber unit.

11. The continuous-flow steam explosion reactor according to any of claims 8 to 10, wherein said scrubber unit is provided with heat exchanging cooling elements to provide cooling of the scrubbing media and recovering heat from the steam explosion.

12. A process for treatment of solid organic material that involves steam explosive disruption and making said material more accessible for further downstream processing, the process comprising:
a) receiving a material stream comprising solid organic material,
b) introducing the stream into a wetting and mixing section and wetting and mixing the solid organic material,
c) transferring the stream from said wetting and mixing section through a loading valve of a loading section into a loading compartment,
d) increasing the pressure in said loading compartment by introducing therein steam or pressurized gas such as pressurized air,
e) releasing material from said loading compartment to a high-pressure retention section while maintaining high pressure and temperature in the high-pressure retention section,
f) closing the loading section from the high-pressure retention section,
g) relieving pressure in the loading section before the loading section is re-loaded from said wetting and mixing section.
h) transferring the material continuously through the high-pressure retention section while subjecting to a high pressure and high temperature,
i) loading a material dose that has been transferred through said high-pressure retention section through a valve to a pressure relief section, releasing pressure from said pressure relief section to attain a steam explosion effect on said material dose,
j) discharging said material dose into a lower pressure discharge section,
k) closing the pressure relief section from the discharge section,
l) increasing pressure in the pressure relief section such that high pressure is maintained in the high-pressure retention section when the pressure relief section is opened towards said high-pressure retention section for re-loading.

13. The process according to claim 12, wherein said releasing pressure in step (i) is achieved by opening a pressure relief valve located in an upper part of said pressure relief section, above its loading valve, allowing escaping gas and steam to exit through said pressure relief valve and a conduit opposite the pressure-relief valve, effectuating steam explosion of the substrate within the pressure relief section while substrate is maintained in the pressure relief section and discharge substrate material subsequently
wherein optionally the discharge of substrate material after pressure relief is assisted by increasing the pressure in the pressure relief chamber before opening said discharge valve to discharge said substrate material into said discharge section.

14. The process according to claim 12 or 13, wherein loading and discharging of the high-pressure retention section in step (c-g) and of the pressure-relief section in step (i-I), pressure relief and material discharge from said compartments, and pressurisation of said loading and pressure relief sections is synchronized such that pressure drop in the high-pressure retention section is minimal during operation.

15. The process according to any of claims 12 to 14, wherein high temperature and high pressure in said high-pressure retention section is achieved by introducing steam into said section and optionally further by introducing pressurized gas such as pressurized air.

16. The process according to any of claims 12 to 15, wherein the stream of material is mixed with alkaline aqueous solution in the wetting and mixing section.

17. The process according to claim 16, wherein the discharge section of the steam explosion reactor is at the same time utilized as scrubber for carbon dioxide and/or other acidic gases with the alkaline steam-exploded substrate and condensed steam providing the scrubbing media.

## Patentansprüche

1. Dampfexplosions-Durchflussreaktor zur Vorbehandlung von organischem Material zum Weiterverarbeiten zu höherwertigen Produkten, Folgendes umfassend:
einen Beladungsabschnitt (1100),
einen Hochdruckretentionsabschnitt (1200),
einen Druckentlastungsabschnitt (1300) und
einen Ausgabeabschnitt (1400),
und wobei der Hochdruckretentionsabschnitt (1200) mindestens einen Förderer mit einstellbarer Geschwindigkeit (1204) zum Transportieren eines Stroms von Quellmaterial durch den Abschnitt umfasst, weiterhin ein Dampfbereitstellungsmittel, das mit einer Dampfquelle verbunden ist, zum Bereitstellen von Dampf in mindestens den Hochdruckretentionsabschnitt (1200) umfasst, wobei der Beladungsabschnitt (1100) eingerichtet ist, um Material bei Umgebungsdruck dem Beladungsabschnitt (1100) vorgeordnet an den Hochdruckretentionsabschnitt (1200) zu transferieren, während hoher Druck und hohe Temperatur in dem Hochdruckretentionsabschnitt (1200) mittels Dampfinjektion in den Beladungsabschnitt (1100) gehalten werden,
**dadurch gekennzeichnet, dass** der Druckentlastungsabschnitt (1300) eingerichtet ist, um Material aus dem Hochdruckretentionsabschnitt (1200) in einen Ausgabeabschnitt (1400) zu transferieren, während hoher Druck in dem Hochdruckretentionsabschnitt (1200) mittels Dampfinjektion in den Druckentlastungsabschnitt (1300) gehalten wird, und
wobei das Dampfinjektionsmittel in dem Beladungsabschnitt (1100) und das Dampfinjektionsmittel in dem Druckentlastungsabschnitt (1300) und Beladen und Entlasten von Abschnitten derartig synchronisiert sind, dass ein Druckabfall in dem Hochdruckretentionsabschnitt **(1200)** minimal ist und hoher Betriebsdruck und hohe Betriebstemperatur beim Betrieb unterhalten werden.

2. Dampfexplosions-Durchflussreaktor nach Anspruch 1, ein Gasbereitstellungsmittel umfassend, das mit einer Druckgasquelle in mindestens einem des Hochdruckretentionsabschnitts (1200), des Beladungsabschnitts (1100) und des Druckentlastungsabschnitts (1300) verbunden ist, um mindestens einen Teil des hohen Drucks durch Druckgasinjektionen zu erzeugen, wobei eine hohe Temperatur durch Dampfinjektionen erzielt wird.

3. Dampfexplosions-Durchflussreaktor nach Anspruch 1 oder 2, wobei der Beladungsabschnitt (1100) mindestens ein Drehdosierventil (1106) und mindestens ein Ausgabedrehventil (1101) umfasst, wobei das Drehdosierventil bemessen und eingerichtet ist, um eine geeignete Substratdosis an das Ausgabedrehventil (1101) zu transferieren, und der Beladungsabschnitt (1100) ein Druckeinstellmittel umfasst, das mit dem Dampfbereitstellungsmittel und/oder dem Gasbereitstellungsmittel gekoppelt ist, um den Druck einer Kammer in dem Ausgabedrehventil (1101) derartig einzustellen, dass der Druck und die Temperatur in dem Hochdruckretentionsabschnitt (1200) im Wesentlichen beibehalten wird, wenn die Kammer Material in den Hochdruckretentionsabschnitt (1200) ausgibt.

4. Dampfexplosions-Durchflussreaktor nach einem der Ansprüche 1 bis 3, wobei der Druckentlastungsabschnitt (1300) mindestens ein Drehdosierventil (1306) und mindestens ein Ausgabedrehventil (1307) umfasst, wobei das Drehdosierventil bemessen und eingerichtet ist, um eine geeignete Substratdosis an das Ausgabedrehventil zu transferieren, und der Druckentlastungsabschnitt ein Druckeinstellmittel umfasst, das mit dem Dampfbereitstellungsmittel und/oder dem Gasbereitstellungsmittel gekoppelt ist, um den Druck und die Temperatur einer Kammer in dem Ausgabedrehventil nach einer Ausgabe derartig einzustellen, dass der Druck in dem Hochdruckretentionsabschnitt und in dem Drehdosierventil im Wesentlichen beibehalten wird, wenn die Kammer in eine Position zum Aufnehmen von Material aus dem Drehdosierventil zurückkehrt.

5. Dampfexplosions-Durchflussreaktor nach einem der Ansprüche 1 bis 3, wobei der Beladungsabschnitt (1100) bzw. der Druckentlastungsabschnitt (1300) mindestens ein Beladeventil und mindestens ein Ausgabeventil und mindestens eine Beladekammer zwischen den Ventilen oder als Teil des Ausgabeventils umfassen, die Beladekammer mit den Dampfinjektionsports und einem optionalen Gasinjektionsmittel verbunden ist und mit einem Druckentlastungsmechanismus versehen ist, um die Kammer vor Ausgabe bzw. Beladung unter Druck zu setzen und den Druck herabsetzen und vor Ausgabe auf eine erwünschte Temperatur zu bringen.

6. Dampfexplosions-Durchflussreaktor nach Anspruch 4 oder 5, wobei der Druckentlastungsabschnitt (1300) ein Druckentlastungsventil (1343) umfasst, das mit einem oberen Abschnitt der Kammer verbunden ist, und wobei das Beladeventil des Druckentlastungsabschnitts unter dem Druckentlastungsventil positioniert ist, und sich eine Entlastungsrohrleitung von dem Druckentlastungsventil zur Aufnahme von entweichendem Dampf und/oder Gas erstreckt, wobei das Ausgabeventil am Boden des Druckentlastungsabschnitts positioniert ist, wobei die Entlastungsrohrleitung vorzugsweise einen Kondensor, einen Ausgabeport und einen optionalen Schalldämpfer umfasst.

7. Dampfexplosions-Durchflussreaktor nach einem der Ansprüche 2 bis 6, wobei das Druckgas mindestens für einen (manche) Abschnitt(e) einen Partialdruck Kohlendioxid enthält.

8. Dampfexplosions-Durchflussreaktor nach einem der vorhergehenden Ansprüche, der zum Betreiben als ein alkalischer Dampfexplosionsreaktor eingerichtet ist,
mindestens eine integrierte Wäschereinheit umfassend, wobei der Ausgabeabschnitt des Dampfexplosionsreaktors gleichzeitig dadurch als die Wäschereinheit dient, dass der Abschnitt derartig eingerichtet ist, dass Ausgabematerial aus dem Dampfexplosionsreaktor als Waschmedium dient, wobei die Wäschereinheit mindestens einen Gaseinlass umfasst, der innerhalb eines unteren Teils der Wäschereinheit bereitgestellt ist, so dass beim Betrieb ein alkalischer Materialstrom aus dem Hochdruckretentionsabschnitt auf einen Gasstrom trifft, um Kohlendioxid-Waschen und/oder Waschen anderer saurer Gaskomponenten aus dem Gasstrom zu unterstützen, gegebenenfalls einen sekundären Wäscher umfassend, der eingerichtet ist, um mit einer flüssigen Fraktion aus der Wäschereinheit als Waschmedium gespeist zu werden.

9. Dampfexplosions-Durchflussreaktor nach Anspruch 8, wobei ein oberer Teil der Wäschereinheit eingerichtet ist, um Lieferung eines Materialstroms aus dem Druckentlastungsabschnitt in einem Winkel in Bezug auf die zentrale Achse der Wäschereinheit aufzunehmen, um den Materialstrom mittels mindestens einer internen Spirale in einer Wirbelstruktur zu führen, und die Wäschereinheit vorzugsweise zwei eingesetzte Spiralen umfasst, die zueinander vertikal versetzt sind, wobei die obere Spirale Abstand zu der Innenwand des Wäschers aufweist, während die untere Spirale keinen Abstand aufweist, was eine partielle Geschwindigkeitskomponente senkrecht zu der primären Wirbelstrukturströmung des Abfallstroms in der Wäschereinheit bewirkt, der durch Material bewirkt wird, das entlang der Innenwand der Wäschereinheit von der oberen Spirale zu der unteren Spirale befördert wird.

10. Dampfexplosions-Durchflussreaktor nach Anspruch 9, wobei die untere Spirale mit einer gewellten Struktur, die Material teilweise zur Mitte des Wäschers führt, und mit einem vorstehenden Kranz an dem Innenrand der Spirale versehen ist, der bewirkt, dass die senkrechte Komponente des Materials verspritzt und zur Mitte der Wäschereinheit dispergiert.

11. Dampfexplosions-Durchflussreaktor nach einem der Ansprüche 8 bis 10, wobei die Wäschereinheit mit Wärme austauschenden Kühlelementen versehen ist, um eine Kühlung der Waschmedien bereitzustellen und Wärme aus der Dampfexplosion zurückzugewinnen.

12. Verfahren zur Behandlung von festem organischem Material, das Dampfexplosionsaufspaltung einbezieht und das Material für weiteres nachgeordnetes Verarbeiten zugänglicher macht, das Verfahren Folgendes umfassend:
a) Aufnehmen eines Materialstroms, der festes organisches Material umfasst,
b) Einführen des Stroms in einen Benetzungs- und Mischabschnitt und Benetzen und Mischen des festen organischen Materials,
c) Transferieren des Stroms aus dem Benetzungs- und Mischabschnitt durch ein Beladeventil eines Beladungsabschnitts in eine Beladungskammer,
d) Erhöhen des Drucks in der Beladungskammer durch Einführen von Dampf oder Druckgas darin, wie beispielsweise Druckluft,
e) Ausgeben von Material aus der Beladungskammer in einen Hochdruckretentionsabschnitt, während hoher Druck und hohe Temperatur in dem Hochdruckretentionsabschnitt beibehalten werden,
f) Schließen des Beladungsabschnitts von dem Hochdruckretentionsabschnitt,
g) Entlasten des Drucks in dem Beladungsabschnitt, bevor der Beladungsabschnitt aus dem Benetzungs- und Mischabschnitt erneut beschickt wird.
h) kontinuierliches Transferieren des Materials durch den Hochdruckretentionsabschnitt, während es einem hohen Druck und einer hohen Temperatur ausgesetzt wird,
i) Beladen einer Materialdosis, die durch den Hochdruckretentionsabschnitt transferiert wurde, durch ein Ventil in einen Druckentlastungsabschnitt, Entlasten des Drucks aus dem Druckentlastungsabschnitt, um eine Dampfexplosionswirkung an der Materialdosis zu erzielen,
j) Ausgeben der Materialdosis in einen Ausgabeabschnitt mit geringerem Druck,
k) Schließen des Druckentlastungsabschnitts von dem Ausgabeabschnitt,
l) Erhöhen des Drucks in dem Druckentlastungsabschnitt, so dass hoher Druck in dem Hochdruckretentionsabschnitt unterhalten wird, wenn der Druckentlastungsabschnitt zu dem Hochdruckretentionsabschnitt hin zum erneuten Beladen geöffnet wird.

13. Verfahren nach Anspruch 12, wobei das Entlasten des Drucks in Schritt (i) durch Öffnen eines Überdruckventils erzielt wird, das in einem oberen Teil des Druckentlastungsabschnitts über seinem Beladeventil angeordnet ist, was entweichendem Gas und Dampf ermöglicht, durch das Druckentlastungsventil und eine Rohrleitung auf der anderen Seite des Druckentlastungsventils auszutreten, wobei eine Dampfexplosion des Substrats in dem Druckentlastungsabschnitt bewirkt wird, während Substrat in dem Druckentlastungsabschnitt gehalten wird und Substratmaterial nachfolgend ausgegeben wird,
wobei gegebenenfalls das Ausgeben von Substratmaterial nach Druckentlastung durch Erhöhen des Drucks in der Druckentlastungskammer vor Öffnen des Ausgabeventils unterstützt wird, um das Substratmaterial in den Ausgabeabschnitt auszugeben.

14. Verfahren nach Anspruch 12 oder 13, wobei Beladen und Entleeren des Hochdruckretentionsabschnitts in Schritt (c-g) und des Druckentlastungsabschnitts in Schritt (i-l), Druckentlastung und Materialausgabe aus den Kammern und Beaufschlagung des Beladungs- und Druckentlastungsabschnitts mit Druck derartig synchronisiert wird, dass ein Druckabfall in dem Hochdruckretentionsabschnitt beim Betrieb minimal ist.

15. Verfahren nach einem der Ansprüche 12 bis 14, wobei hohe Temperatur und hoher Druck in dem Hochdruckretentionsabschnitt durch Einführen von Dampf in den Abschnitt und gegebenenfalls ferner durch Einführen von Druckgas, wie beispielsweise Druckluft, erzielt wird.

16. Verfahren nach einem der Ansprüche 12 bis 15, wobei der Materialstrom in dem Benetzungs- und Mischabschnitt mit alkalischer wässriger Lösung vermischt wird.

17. Verfahren nach Anspruch 16, wobei der Ausgabeabschnitt des Dampfexplosionsreaktors gleichzeitig als Wäscher für Kohlendioxid und/oder andere saure Gase eingesetzt wird, wobei das mit alkalischem Dampf explodierte Substrat und kondensierter Dampf die Waschmedien bereitstellen.

## Revendications

1. Réacteur à explosion de vapeur à écoulement continu pour le prétraitement de matière organique pour la transformation supplémentaire en produits à valeur ajoutée, comprenant :
une section de chargement (1100),
une section de conservation de haute pression (1200),
une section de détente de pression (1300), et
une section d'évacuation (1400),
et dans lequel ladite section de conservation de haute pression (1200) comprend au moins un transporteur à vitesse ajustable (1204) pour transporter un courant de matière source à travers ladite section, comprenant en outre des moyens de fourniture de vapeur raccordés à une source de vapeur, pour fournir de la vapeur dans au moins ladite section de conservation de haute pression (1200), dans lequel ladite section de chargement (1100) est configurée pour transférer une matière, à pression ambiante, en amont de la section de chargement (1100), à ladite section de conservation de haute pression (1200) tout en conservant de hautes pression et température dans la section de conservation de haute pression (1200) au moyen d'injection de vapeur dans ladite section de chargement (1100),
**caractérisé en ce que** ladite section de détente de pression (1300) est configurée pour transférer une matière, depuis ladite section de conservation de haute pression (1200), à une section d'évacuation (1400), tout en conservant une haute pression dans la section de conservation de haute pression (1200) au moyen d'injection de vapeur dans ladite section de détente de pression (1300), et
dans lequel lesdits moyens d'injection de vapeur dans ladite section de chargement (1100) et lesdits moyens d'injection de vapeur dans ladite section de détente de pression (1300) et le chargement et la libération depuis des sections sont synchronisés de telle sorte qu'une chute de pression dans la section de conservation de haute pression (1200) soit minimale et de hautes pression et température de fonctionnement soient maintenues durant le fonctionnement.

2. Réacteur à explosion de vapeur à écoulement continu selon la revendication 1, comprenant des moyens de fourniture de gaz raccordés à une source de gaz sous pression, entrant dans au moins une de ladite section de conservation de haute pression (1200), de ladite section de chargement (1100) et de ladite section de détente de pression (1300), pour générer au moins une partie de haute pression par le biais d'injections gaz sous pression, dans lequel la haute température est obtenue par le biais d'injections de vapeur.

3. Réacteur à explosion de vapeur à écoulement continu selon la revendication 1 ou 2, dans lequel ladite section de chargement (1100) comprend au moins une soupape de dosage rotative (1106) et au moins une soupape d'évacuation rotative (1101), dans lequel ladite soupape de dosage rotative est dimensionnée et configurée pour transférer une dose appropriée de substrat à la soupape d'évacuation rotative (1101), et ladite section de chargement (1100) comprend des moyens d'ajustement de pression accouplés auxdits moyens de fourniture de vapeur et/ou auxdits moyens de fourniture de gaz, pour ajuster la pression d'un compartiment dans ladite soupape d'évacuation rotative (1101) de telle sorte que la pression et la température à l'intérieur de ladite section de conservation de haute pression (1200) soient sensiblement maintenues lorsque ledit compartiment évacue une matière dans ladite section de conservation de haute pression (1200).

4. Réacteur à explosion de vapeur à écoulement continu selon l'une quelconque des revendications 1 à 3, dans lequel ladite section de détente de pression (1300) comprend au moins une soupape de dosage rotative (1306) et au moins une soupape d'évacuation rotative (1307), dans lequel ladite soupape de dosage rotative est dimensionnée et configurée pour transférer une dose appropriée de substrat à la soupape d'évacuation rotative, et ladite section de détente de pression comprend des moyens d'ajustement de pression accouplés auxdits moyens de fourniture de vapeur et/ou auxdits moyens de fourniture de gaz, pour ajuster la pression et la température d'un compartiment dans ladite soupape d'évacuation rotative après l'évacuation, de telle sorte que la pression à l'intérieur de ladite section de conservation de haute pression et de ladite soupape de dosage rotative soit sensiblement maintenue lorsque ledit compartiment retourne à une position pour recevoir une matière à partir de ladite soupape de dosage rotative.

5. Réacteur à explosion de vapeur à écoulement continu selon l'une quelconque des revendications 1 à 3, dans lequel ladite section de chargement (1100) et/ou ladite section de détente de pression (1300) comprennent respectivement au moins une soupape de chargement et au moins une soupape d'évacuation, et au moins une chambre de chargement entre lesdites soupapes ou faisant partie de ladite soupape d'évacuation, ladite chambre de chargement est accouplée auxdits orifices d'injection de vapeur et à des moyens optionnels d'injection de gaz et pourvue d'un mécanisme de détente de pression, pour mettre sous pression ladite chambre, et réduire la pression de cette dernière, avant l'évacuation et le chargement, respectivement, et pour amener à une température souhaitée avant l'évacuation.

6. Réacteur à explosion de vapeur à écoulement continu selon la revendication 4 ou 5, dans lequel ladite section de détente de pression (1300) comprend une soupape de détente de pression (1343) raccordée à une portion supérieure de ladite chambre et dans lequel ladite soupape de chargement de la section de détente de pression est positionnée en dessous de ladite soupape de détente de pression, et un conduit de détente s'étendant depuis ladite soupape de détente de pression pour recevoir de la vapeur et/ou du gaz s'échappant, ladite soupape d'évacuation étant positionnée au fond de la section de détente de pression, dans lequel ledit conduit de détente comprend de préférence un condenseur, un orifice d'évacuation et un silencieux optionnel.

7. Réacteur à explosion de vapeur à écoulement continu selon l'une quelconque des revendications 2 à 6, dans lequel ledit gaz sous pression comprend, au moins pour certaine(s) section(s), une pression partielle de dioxyde de carbone.

8. Réacteur à explosion de vapeur à écoulement continu selon l'une quelconque des revendications précédentes, qui est configuré pour fonctionner en tant que réacteur à explosion de vapeur alcaline, comprenant au moins une unité épuratrice intégrée, dans lequel ladite section d'évacuation du réacteur à explosion de vapeur sert, en même temps, de dite unité épuratrice, en ce que la section est configurée de telle sorte qu'une matière d'évacuation provenant du réacteur à explosion de vapeur serve de substance d'épuration, dans lequel l'unité épuratrice comprend au moins une entrée de gaz prévue à l'intérieur d'une partie inférieure de l'unité épuratrice, de telle sorte que, lors du fonctionnement, un courant de matière alcaline provenant de la section de conservation de haute pression rejoigne un courant de gaz pour faciliter l'épuration de dioxyde de carbone et/ou l'épuration d'autres composants gazeux acides à partir dudit courant de gaz, optionnellement comprenant un épurateur secondaire qui est configuré pour être alimenté en une fraction liquide provenant de ladite unité épuratrice en tant que substance d'épuration.

9. Réacteur à explosion de vapeur à écoulement continu selon la revendication 8, dans lequel une partie supérieure de l'unité épuratrice est adaptée pour recevoir une distribution d'un courant de matière, à partir de la section de détente de pression, selon un angle par rapport à l'axe central de l'unité épuratrice, pour diriger ledit courant de matière en une configuration cyclonique au moyen d'au moins une spirale interne, et, de préférence, l'unité épuratrice comprend deux spirales insérées qui sont verticalement décalées l'une par rapport à l'autre, la spiral supérieure ayant espace libre par rapport à la paroi intérieure de l'épurateur alors que la spiral inférieure n'a aucun espace libre, ce qui entraîne une composante de vitesse partielle perpendiculaire à l'écoulement en configuration cyclonique primaire du courant de déchets à l'intérieur de l'unité d'épuration, entraîné par une matière transportée depuis la spiral supérieure jusqu'à la spiral inférieure le long de la paroi intérieure de l'unité épuratrice.

10. Réacteur à explosion de vapeur à écoulement continu selon la revendication 9, dans lequel ladite spirale inférieure est pourvue d'une configuration ondulée guidant partiellement une matière vers le centre de l'épurateur et d'un pourtour saillant au bord intérieur de la spirale faisant en sorte que la composante perpendiculaire de la matière soit aspergée et se disperse vers le centre de l'unité épuratrice.

11. Réacteur à explosion de vapeur à écoulement continu selon l'une quelconque des revendications 8 à 10, dans lequel ladite unité épuratrice est pourvue d'éléments de refroidissement à échange de chaleur pour fournir un refroidissement de la substance d'épuration et récupérer de la chaleur à partir de l'explosion de vapeur.

12. Procédé pour traitement de matière organique solide qui implique la perturbation par explosion de vapeur et l'accroissement de l'accessibilité à ladite matière pour une transformation en aval supplémentaire, le procédé comprenant :
a) la réception d'un courant de matière comprenant une matière organique solide,
b) l'introduction du courant dans une section de mouillage et de mélangeage et le mouillage et le mélangeage de la matière organique solide,
c) le transfert du courant, depuis ladite section de mouillage et de mélangeage, par le biais d'une soupape de chargement d'une section de chargement, dans un compartiment de chargement,
d) l'augmentation de la pression dans ledit compartiment de chargement en introduisant dans celui-ci de la vapeur, ou un gaz sous pression tel que de l'air sous pression,
e) la libération de matière, depuis ledit compartiment de chargement, dans une section de conservation de haute pression tout en maintenant de hautes pression et température dans la section de conservation de haute pression,
f) la fermeture de la section de chargement par rapport à la section de conservation de haute pression,
g) la détente de pression dans la section de chargement avant que la section de chargement soit rechargée à partir de ladite section de mouillage et de mélangeage,
h) le transfert de la matière en continu à travers la section de conservation de haute pression tout en étant soumise à une haute pression et une haute température,
i) le chargement d'une dose de matière qui a été transférée, à travers ladite section de conservation de haute pression, par le biais d'une soupape, à une section de détente de pression, la libération de pression, à partir de ladite section de détente de pression, pour atteindre un effet d'explosion de vapeur sur ladite dose de matière,
j) l'évacuation de ladite dose de matière, dans une section d'évacuation de pression inférieure,
k) la fermeture de la section de détente de pression par rapport à la section d'évacuation,
l) l'augmentation de pression dans la section de détente de pression de telle sorte qu'une haute pression soit maintenue dans la section de conservation de haute pression lorsque la section de détente de pression est ouverte vers ladite section de conservation de haute pression pour le rechargement.

13. Procédé selon la revendication 12, dans lequel ladite libération de pression dans l'étape (i) est obtenue en ouvrant une soupape de détente de pression située dans une partie supérieure de ladite section de détente de pression, au-dessus de sa soupape de chargement, permettant à du gaz et de la vapeur s'échappant de sortir à travers ladite soupape de détente de pression et un conduit en face de la soupape de détente de pression, entraînant une explosion de vapeur du substrat à l'intérieur de la section de détente de pression alors que le substrat est maintenu dans la section de détente de pression et l'évacuation d'une matière de substrat par la suite,
dans lequel, optionnellement, l'évacuation de matière de substrat après la détente de pression est assistée en augmentant la pression dans la chambre de détente de pression avant d'ouvrir ladite soupape d'évacuation pour évacuer ladite matière de substrat dans ladite section d'évacuation.

14. Procédé selon la revendication 12 ou 13, dans lequel le chargement et l'évacuation de la section de conservation de haute pression dans l'étape (c-g) et de la section de détente de pression dans l'étape (i-1), la détente de pression et l'évacuation de matière à partir desdits compartiments, et la mise sous pression desdites sections de chargement et de détente de pression sont synchronisés de telle sorte qu'une chute de pression dans la section de conservation de haute pression soit minimale durant le fonctionnement.

15. Procédé selon l'une quelconque des revendications 12 à 14, dans lequel la haute température et la haute pression dans ladite section de conservation de haute pression sont obtenues en introduisant de la vapeur dans ladite section et, optionnellement, en outre, en introduisant un gaz sous pression, tel que de l'air sous pression.

16. Procédé selon l'une quelconque des revendications 12 à 15, dans lequel le courant de matière est mélangé avec une solution aqueuse alcaline dans la section de mouillage et de mélangeage.

17. Procédé selon la revendication 16, dans lequel la section d'évacuation du réacteur à explosion de vapeur est en même temps utilisée en tant qu'épurateur pour dioxyde de carbone et/ou autres gaz acides, le substrat, ayant subi une explosion de vapeur alcaline, et de la vapeur condensée fournissant les substances d'épuration.
